# EUROPÄISCHE PATENTANMELDUNG

(11) **EP 4 563 210 A1**
(43) Veröffentlichungstag der Anmeldung: **04.06.2025**
(21) Anmeldenummer: 24213339.5
(22) Anmeldetag: 15.11.2024
(51) Int. Cl.: B01D 3/42, C10G 7/12, B01D 3/14, C07C 7/04

(54) **ENERGIEEFFIZIENTES DESTILLATIONSVERFAHREN MIT ZUFLUSSVARIATION**

(30) Priorität: 29.11.2023 EP 23213043
(71) Anmelder: Evonik Oxeno GmbH & Co. KG, 45772 Marl (DE)
(72) Erfinder: Paul, Niklas, 45770 Marl (DE); Rix, Armin Matthias, 45770 Marl (DE); Six, Tanita Valèrie, 44309 Dortmund (DE); Steenweg, Tanja, 44139 Dortmund (DE); Wessner, Lea, 44139 Dortmund (DE); Oldemeyer, Martin, 45721 Haltern am See (DE)
(74) Vertreter: Evonik Patent Association

(57) **Zusammenfassung**

Gegenstand der Erfindung ist ein Verfahren zur Destillation eines Raffinat-2-Stroms als Feedstrom in mindestens zwei Destillationskolonnen DK1 und DK2, wobei zumindest die Destillationskolonne DK1 zwei unterschiedliche Einlässe für den Feedstrom aufweist. Das Verfahren zeichnet sich dadurch aus, dass der Feedstrom vor dem Einleiten auf seine Zusammensetzung analysiert wird; und in Abhängigkeit von der Zusammensetzung des Feedstroms einer der mindestens zwei Einlässe für die Zuleitung des Feedstroms gewählt wird.

## Beschreibung

Die vorliegende Erfindung betrifft ein Verfahren zur Destillation eines Raffinat-2-Stroms als Feedstrom in mindestens zwei Destillationskolonnen DK1 und DK2, wobei zumindest die Destillationskolonne DK1 zwei unterschiedliche Einlässe für den Feedstrom aufweist. Das Verfahren zeichnet sich dadurch aus, dass der Feedstrom vor dem Einleiten auf seine Zusammensetzung analysiert wird; und in Abhängigkeit von der Zusammensetzung des Feedstroms einer der mindestens zwei Einlässe für die Zuleitung des Feedstroms gewählt wird.

Die Destillation von Kohlenwasserstoffströmen ist eine bewährte Technik, um Substanzen aus Stoffgemischen abzutrennen. Destillationsverfahren sind in der chemischen Industrie deshalb bei der Produktion vieler chemischer Stoffe unverzichtbar. Beispiele dafür sind die Destillation von Feedströmen zur Abtrennung leicht- oder schwersiedenden Komponenten vor einer chemischen Reaktion oder die Destillation von Rohproduktgemischen zur Abtrennung von Edukten sowie leicht- und/oder schwersiedenden Nebenprodukten.

Die bei der Destillation eingesetzten Kohlenwasserstoffströme unterliegen in aller Regel natürlichen und/oder produktionsbedingten Schwankungen in der Zusammensetzung. Das bedeutet, dass die Anteile der Komponenten in den Kohlenwasserstoffströmen größer oder kleiner werden, neue Komponenten hinzukommen und/oder andere Komponenten wegfallen. Dazu gehören beispielsweise Stoffströme in petrochemischen Produktionsanlagen. Diese Kohlenwasserstoffströme sind insbesondere C4-Kohlenwasserstoffströme aus Steamcrackern, FCC-C4-Ströme, Produktströme aus der MTBE-Synthese (MTBE = Methyl-tert.-butyl-ether), Raffinat-2-Ströme oder Produktströme aus der Oligomerisierung.

C4-Kohlenwasserstoffströme bestehen im Wesentlichen aus Butadien, Isobuten, 1-Buten, den beiden 2-Butenen, Isobutan und n-Butan. Übliche weltweit ausgeübte Aufarbeitungsverfahren für solche C4-Kohlenwasserstoffströme umfassen folgende Schritte: Zuerst wird der größte Teil des Butadiens entfernt. Zurück bleibt in beiden Fällen ein Kohlenwasserstoffgemisch, das neben den gesättigten Kohlenwasserstoffen n-Butan und Isobutan die Olefine Isobuten, 1-Buten und 2-Butene enthält und das als Raffinat 1 bezeichnet wird. Ein möglicher Weg, das Isobuten aus diesem Gemisch zu entfernen, ist die Reaktion mit Methanol zu MTBE. Zurück bleiben die gesättigten Kohlenwasserstoffe, lineare Butene und gegebenenfalls eine Restmenge an Isobuten. Das nach Entfernen des Butadiens und Isobuten erhaltene Gemisch wird als Raffinat 2 bezeichnet.

Schwankende Zusammensetzungen der Anteile der einzelnen Komponenten in den Kohlenwasserstoffströmen und insbesondere im Raffinat 2 können dazu führen, dass die Trennaufgabe nicht mehr ausreichend bewältigt werden kann oder höhere Energiemengen für die Bewältigung der Trennaufgabe eingesetzt werden müssen.

Die Aufgabe der vorliegenden Erfindung war deshalb eine Verbesserung bekannter Destillationsverfahren. Schwankende Zusammensetzungen der Anteile der Komponenten im Raffinat 2 sollten dabei mit möglichst wenig Aufwand abgefangen werden, während die Trennaufgabe möglichst gleichbleibend bewältigt wird.

Diese Aufgabe wird durch die in Anspruch 1 vorgeschlagene Ausführung des Verfahrens gelöst. Bevorzugte Ausführungsformen sind in den Unteransprüchen angegeben. Das erfindungsgemäße Verfahren ist ein Verfahren zur Destillation eines Raffinat-2-Stroms als Feedstrom, in einer Abtrenneinheit, die mindestens zwei Destillationskolonnen DK1 und DK2 umfasst, wobei
die Destillationskolonne DK1 und die DK2 jeweils einen Kopf am oberen Ende der Kolonne und einen Sumpf am unteren Ende der Kolonne umfassen, wobei am Kopf jeweils ein Brüdenstrom und am Sumpf jeweils ein Sumpfstrom anfallen und wobei die Destillationskolonne DK2 mit einem Teil des Brüdenstroms aus der DK2 gespeist wird;
die Brüdenströme von DK1 und DK2 zumindest teilweise kondensiert werden und die dabei anfallende Kondensationsenergie von mindestens einem der Brüdenströme von DK1 oder DK2 mittels Brüdenverdichtung oder mittels einer Wärmepumpe zur Beheizung von zumindest einer der Destillationskolonnen DK1 oder DK2 eingesetzt werden;
zumindest die Destillationskolonne DK1 mindestens zwei unterschiedliche Einlässe für den Feedstrom aufweist, wobei die Einlässe vom Kopf der Destillationskolonne betrachtet unterhalb voneinander angeordnet sind;
der Feedstrom vor dem Einleiten auf seine Zusammensetzung analysiert wird; und

in Abhängigkeit von der Zusammensetzung des Feedstroms einer der mindestens zwei Einlässe für die Zufuhr des Feedstroms gewählt wird.

Ein Vorteil des erfindungsgemäßen Verfahrens ist, dass durch die Wahl eines geeigneten Einlasses in die Destillationskolonne eine möglichst optimale Trennung des eingesetzten Kohlenwasserstoffstroms, des Raffinat-2-Stroms, erfolgt und gleichzeitig Energie eingespart werden kann.

Der Feedstrom, der der erfindungsgemäßen Destillation zugeführt wird, ist ein Kohlenwasserstoffstrom. Der im erfindungsgemäßen Destillationsverfahren eingesetzte Feedstrom ist ein Raffinat-2-Strom. Ein Raffinat-2-Strom enthält zumindest 1-Buten, 2-Buten, n-Butan und Isobutan. Raffinat-2-Ströme können im weiteren Verlauf der petrochemischen Prozessverbünde einer 1-Butenabtrennung unterworfen werden. Dabei wird 1-Buten destillativ aus dem Raffinat 2 abgetrennt und es entsteht ein Raffinat 3. Diese 1-Butenabtrennung wird vorzugsweise nach dem erfindungsgemäßen Destillationsverfahren durchgeführt. Die 1-Butenabtrennung wird übrigens in aller Regel nicht vollumfänglich durchgeführt. Es kann also 1-Buten im Raffinat 3 verbleiben.

Der in dem erfindungsgemäßen Destillationsverfahren eingesetzte Kohlenwasserstoffstrom ist ein Raffinat-2-Strom, der zumindest 1-Buten, 2-Buten, n-Butan und Isobutan enthält. Aus Raffinat-2-Strömen würde bei der Destillation das 1-Buten zumindest teilweise abgetrennt werden.

Vorzugsweise enthält das eingesetzte Raffinat 2 weniger als 2500 ppm, vorzugsweise weniger als 1000 ppm Isobuten, besonders bevorzugt weniger als 500 ppm Isobuten. Weiterhin bevorzugt enthält das im Verfahren der vorliegenden Erfindung eingesetzte Raffinat 2 weniger als 4 Gew.-% mehrfach ungesättigte C4-Kohlenwasserstoffe. In einer besonders bevorzugten Ausführungsform sollte die Konzentration der mehrfach ungesättigten C4-Kohlenwasserstoffe weniger als 500 ppm betragen. Sollten die Ströme höhere Mengen an Butadien enthalten, könnte vorher eine Selektivhydrierung durchgeführt werden, bei der das Butadien zu Butenen und/oder Butanen umgesetzt wird. Entsprechende Verfahren sind dem Fachmann bekannt.

Der eingesetzte Raffinat-2-Strom kann darüber hinaus gewisse Mengen an Wasser enthalten, insbesondere in einer Menge von 150 bis 4000 ppm. Es ist bevorzugt, dass das Wasser durch die Destillation zumindest teilweise abgetrennt wird. Das Wasser wird sich dabei in der Destillationskolonne im anfallenden Brüdenstrom anreichern und nach Kondensation als zweite flüssige Phase anfallen, die über Euter in den Destillatbehältern der Destillationskolonne abgetrennt werden kann. Das Sumpfprodukt der Destillationskolonne zeichnet sich durch sehr niedrige Gehalte an Butadien und Wasser, bevorzugt je unter 100 ppm, besonders bevorzugt unter 5 ppm aus.

Das erfindungsgemäße Verfahren wird in einer Abtrenneinheit durchgeführt, die mindestens zwei Destillationskolonnen DK1 und DK2 umfasst. Die Destillationskolonnen können jedwede bekannte Destillationskolonne sein, die für das entsprechende Trennverfahren geeignet ist. Die Destillationskolonnen DK1 und DK2 sind so aufgebaut, dass sie jeweils am oberen Ende der Kolonne einen Kopf und am unteren Ende der Kolonne einen Sumpf umfasst. Zumindest die Destillationskolonne DK1 weist mindestens zwei unterschiedliche Einlässe auf, über die der eingesetzte Feedstrom, also das Raffinat 2, in die Destillationskolonne DK1 gelangen kann. Die mindestens zwei Einlässe sind dabei vom Kopf der Kolonne, also vom oberen Ende der Kolonne betrachtet, unterhalb voneinander angeordnet. Die mindestens zwei Einlässe befinden sich also nicht auf der gleichen Höhe, sondern sind räumlich und von unten betrachtet auf einer unterschiedlichen Höhe angeordnet. Es ist auch möglich, dass die Destillationskolonne DK2 mindestens zwei unterschiedliche Einlässe aufweist, über die der Strom aus der DK1 in die Destillationskolonne DK2 gelangen kann.

Es versteht sich, dass die Destillationskolonne DK1 oder DK2 für das erfindungsgemäße Verfahren auch mehr als zwei, also drei, vier, fünf oder mehr Einlässe aufweisen kann, über die der jeweilige Strom je nach seiner Zusammensetzung in die Destillationskolonne geleitet wird.

Die für die Trennaufgabe benötigte Wärmeenergie wird in Destillationskolonnen üblicherweise über mindestens einen Sumpfverdampfer eingebracht. Durch den Sumpfverdampfer fließt dabei vorzugsweise ein Strom, der am unteren Ende, also im oder am Sumpf der Destillationskolonne entnommen und nach Durchlaufen des Sumpfverdampfers wieder zur Destillationskolonne zurückgeführt wird. Der Strom wird beim Durchlaufen des Sumpfverdampfers erhitzt.

Als "Sumpfverdampfer" werden erfindungsgemäß Verdampfer bezeichnet, die den Sumpf der jeweiligen Destillationskolonne beheizen. Ein derartiger Sumpfverdampfer ist üblicherweise außerhalb der jeweiligen Destillationskolonne angeordnet. Da in Sumpfverdampfern Energie, insbesondere Wärme, von einem Strom auf einen anderen übertragen wird, sind sie Wärmeüberträger. Über einen Abzug aus dem Sumpf der Destillationskolonne wird der (zumindest teilweise) zu verdampfende Strom abgezogen und dem Sumpfverdampfer zugeführt. Über mindestens einen Zulauf wird der verdampfte Strom gegebenenfalls mit einem Restanteil Flüssigkeit wieder in die jeweilige Destillationskolonne im Bereich des Sumpfs zurückgeführt.

Geeignete Verdampfer, die als Sumpfverdampfer eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Als Wärmeübertrager für den Verdampfer wird bei Naturumlaufverdampfern und Zwangsumlaufverdampfern üblicherweise ein Rohrbündel oder Plattenapparat eingesetzt. Neben den genannten kann aber auch jede beliebige andere, dem Fachmann bekannte Verdampferbauart, die sich zum Einsatz an einer Destillationskolonne eignet, eingesetzt werden.

Die mindestens zwei Destillationskolonnen weisen vorzugsweise außerdem eine Vielzahl von Einbauten, wie Füllkörperschüttungen, (strukturierten) Packungen oder Böden auf. Diese Einbauten sorgen dafür, dass der in der Destillationskolonne aufsteigende Dampf und die herabfließende Flüssigkeit sich ausreichend kontaktieren und verbessern den Stoff- und Wärmeaustausch. Als Böden werden üblicherweise Glockenböden, Siebböden, Ventilböden mit festen oder beweglichen Ventilen, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel, SuperRinge/SuperRinge Plus oder Intalox^{®}-Sättel verwendet. Strukturierte Packungen werden zum Beispiel unter dem Handelsnamen Mellapak^{®} der Firma Sulzer vertrieben. Diese und andere geeignete Einbauten sind dem Fachmann bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der oder den Destillationskolonnen möglichst gering bleibt und somit die mechanische Leistung des Verdichters und die Temperatur des zu verdampfenden Raffinat-2-Stroms gering bleibt.

In einer besonders bevorzugten Ausführungsform der vorliegenden Erfindung umfasst die Destillationskolonne DK1 Einbauten, vorzugsweise zwischen 2 und 300 Einbauten, weiterhin bevorzugt zwischen 2 und 250 Einbauten, besonders bevorzugt 2 bis 220 Einbauten. Die Einbauten sind innerhalb der Destillationskolonne DK1 vom Kopf der Destillationskolonne betrachtet unterhalb voneinander angeordnet. In einer bevorzugten Ausführungsform der vorliegenden Erfindung befinden sich die Einlässe jeweils auf Höhe mindestens einer der Einbauten. Da die Einlässe untereinander angeordnet sind, können die Einlässe grundsätzlich auch auf einer unterschiedlichen Höhe bezogen auf eine der Einbauten angeordnet sein. Es ist jedoch besonders bevorzugt, dass die mindestens zwei Einlässe auf Höhe unterschiedlicher Einbauten vorhanden sind.

In einer weiterhin besonders bevorzugten Ausführungsform der vorliegenden Erfindung umfasst die Destillationskolonne DK2 Einbauten, vorzugsweise zwischen 2 und 300 Einbauten, weiterhin bevorzugt zwischen 2 und 250 Einbauten, besonders bevorzugt 2 bis 220 Einbauten. Die Einbauten sind innerhalb der Destillationskolonne DK2 vom Kopf der Destillationskolonne betrachtet unterhalb voneinander angeordnet. In einer bevorzugten Ausführungsform der vorliegenden Erfindung befinden sich die Einlässe jeweils auf Höhe mindestens einer der Einbauten. Da die Einlässe untereinander angeordnet sind, können die Einlässe grundsätzlich auch auf einer unterschiedlichen Höhe bezogen auf eine der Einbauten angeordnet sein. Es ist jedoch besonders bevorzugt, dass die mindestens zwei Einlässe auf Höhe unterschiedlicher Einbauten vorhanden sind.

Die Einbauten in Destillationskolonnen definieren sich über die von ihnen erzeugten theoretischen Trennstufen. Über die Anzahl der theoretischen Trennstufen werden Destillationskolonnen in der Regel ausgelegt. Bei Böden als Einbauten ergibt sich die Anzahl der theoretischen Trennstufen durch Multiplikation mit dem Bodenwirkungsgrad (Anzahl theoretische Trennstufen = Anzahl Böden * Bodenwirkungsgrad). Bei Füllkörperschüttungen oder (strukturierten) Packungen ergibt sich die Anzahl der theoretischen Trennstufen über den HETP-Wert (height equivalent for one theoretical plate) durch Multiplikation mit der Höhe der eingesetzten Füllkörperschüttung oder (strukturierte) Packung (Anzahl theoretische Trennstufen = HETP-Wert * Höhe Füllkörperschüttung oder (strukturierte) Packung). Der HETP-Wert kann anhand des zu trennenden Stoffgemisches und der eingesetzten Füllkörperschüttung oder (strukturierten) Packung bestimmt werden. Üblicherweise ergeben sich mehrere theoretische Trennstufen aus einer Füllkörperschüttung oder einer (strukturierten) Packung, also einer der Einbauten.

Es sollte klar sein, dass die Abtrenneinheit eine oder mehrere weitere Destillationskolonnen umfassen kann. Ob eine oder mehrere weitere Destillationskolonne(n) in der Abtrenneinheit vorhanden sind, hängt von der zu bewältigenden Trennaufgabe ab. Sofern auch im Strom zu der oder den weiteren Destillationskolonne(n) Schwankungen in der Feedzusammensetzung zu erwarten sind, wäre die Variation des gewählten Einlasses anhand der Zusammensetzung des jeweiligen Stroms auch hier anzuwenden. Die eine oder mehreren Destillationskolonne(n) können ebenso aufgebaut sein wie die bereits beschriebene Destillationskolonne, d. h. einen Sumpf und einen Kopf aufweisen, einen Sumpfverdampfer zum Eintrag der Wärmeenergie nutzen und eine Vielzahl an Einbauten wie Füllkörperschüttungen, (strukturierte) Packungen oder Böden umfassen.

Der erste Schritt des erfindungsgemäßen Verfahrens ist, dass der Feedstrom, also das Raffinat 2, vor dem Einleiten auf seine Zusammensetzung analysiert wird. Damit ist gemeint, dass zumindest die (prozentualen) Anteile, Konzentrationen oder Mengen der Komponente(n) des Raffinat-2-Stroms bestimmt werden, die für die Wahl des Einlasses für die Zuleitung des Kohlenwasserstoffstroms relevant sind. In einer bevorzugten Ausführungsform werden jeweils die Komponenten der charakteristischen Trennungen bestimmt, im vorliegenden Fall, bei Einsatz eines Raffinat-2-Stroms, ist es bevorzugt, dass der n-Butananteil und/oder der Isobutananteil bestimmt werden. Die Analyse der Zusammensetzung kann nach allen bekannten Analyseverfahren durchgeführt werden. Bevorzugte Analyseverfahren für das erfindungsgemäße Verfahren sind die Raman-Spektroskopie und die Gaschromatographie. Die Analyse der Zusammensetzung des Raffinat-2-Stroms kann grundsätzlich online erfolgen, d. h. dass die Messung innerhalb des Verfahrens erfolgt. Andererseits kann die Messung auch so erfolgen, dass an einer geeigneten Stelle eine Probe gezogen und diese Probe dann außerhalb des Verfahrens analysiert wird.

In Abhängigkeit von der Zusammensetzung des Raffinat-2-Stroms bzw. des der DK2 zugeführten Stroms wird anschließend einer der mindestens zwei Einlässe für die Zuleitung des jeweiligen Stroms gewählt. Das bedeutet, dass entweder der - bezogen auf die mindestens zwei Einlässe - höher oder niedriger gelegene Einlass der mindestens zwei Einlässe als Zulauf für die Destillationskolonne fungiert, über den der jeweilige Strom, also z. B. der Raffinat-2-Strom, in die Destillationskolonne geleitet wird. Sind mehr als zwei Einlässe vorhanden gilt das entsprechend, wobei nicht zwangsläufig der höchst gelegene oder der tiefst gelegene Einlass gewählt werden muss, sondern auch der oder die dazwischen liegenden Einlässe.

Die Rohrleitungen zu den mindestens zwei Einlässen der Destillationskolonne bei der erfindungsgemäßen Destillation weisen vorzugsweise geeignete Verschlussvorrichtungen auf, um die Rohrleitung zu jedem Einlass unabhängig voneinander verschließen und öffnen zu können. Entsprechende Verschlussvorrichtungen sind dem Fachmann geläufig, beispielsweise geeignete Arten von Ventilen.

Das erfindungsgemäße Verfahren kann grundsätzlich kontinuierlich oder in Batch-Fahrweise betrieben werden. Bevorzugt handelt es sich bei dem erfindungsgemäßen Verfahren um ein kontinuierlich betriebenes Verfahren.

Sowohl bei kontinuierlich betriebenen Verfahren als auch bei in Batch-Fahrweise betriebenen Verfahren kann sich während des Verfahrens die Zusammensetzung des eingesetzten Stroms ändern. Industriell eingesetzte Stoffströme, hier das Raffinat 2, unterliegen dabei regelmäßig gewissen Schwankungen. Nicht immer ist diese Änderung in der Zusammensetzung so groß, dass dies einen Einfluss auf das betreffende Destillationsverfahren hat. Es ist aber auch möglich, dass sich während des erfindungsgemäßen Verfahrens die Zusammensetzung des jeweiligen Stroms ändert und deshalb auf einen anderen Einlass umgeschaltet werden muss. Das bedeutet, dass die Zusammensetzung des jeweils eingesetzten Stroms nicht nur einmal analysiert wird, sondern in vorab definierten zeitlichen Abständen kontinuierlich während des gesamten Verfahrens. Der Abstand zwischen zwei Analysemessungen kann dabei je nach Art des eingesetzten Kohlenwasserstoffstroms variiert werden. Grundsätzlich können zwischen zwei Analysemessungen 10 bis 59 Sekunden, 1 bis 59 Minuten, 1 bis 23 Stunden oder 1 bis mehrere Tage liegen.

Sofern eine Umschaltung von dem einen auf den anderen der mindestens zwei Einlässe erforderlich wird, kann die Umschaltung des jeweiligen Stroms auf einen anderen Einlass automatisch oder manuell erfolgen. Bei der Umschaltung wird der eine Einlass und/oder die Leitung zum Einlass verschlossen und der andere Einlass und/oder die Leitung zum Einlass geöffnet.

Sofern die Umschaltung auf einen anderen Einlass automatisch erfolgt, kann die Umschaltung so erfolgen, dass die Analyseergebnisse mittels einer Steuereinheit, beispielsweise mittels eines Computers ausgewertet werden und bei Über- oder Unterschreiten eines Grenzwerts in der Zusammensetzung des jeweiligen Stroms die Umschaltung auf einen anderen Einlass erfolgt. Eine derartige Automatisierung unter Zuhilfenahme einer computergestützten Auswertung ist recht einfach zu realisieren, da es sich um einen Vergleich zwischen Grenzwert und Messwert handelt. Weiterhin wird der menschliche Fehler möglichst vermieden und die Umschaltung kann schneller erfolgen. Als Grenzwert eignen sich alle messbaren Parameter in Zusammenhang mit der Zusammensetzung des Stroms oder seiner Komponenten, also beispielsweise die bereits erwähnten (prozentualen) Anteile, Konzentrationen oder Mengen der Komponente(n) im Strom.

Es ist im Rahmen des vorliegenden Verfahrens bevorzugt, wenn mindestens ein der zwei Destillationskolonnen DK1 Und DK2 mit Rücklauf betrieben wird, vorzugsweise beide Destillationskolonnen DK1 und DK2 mit Rücklauf betrieben werden. Rücklauf bedeutet, dass der am oberen Ende der Destillationskolonne entnommene Brüdenstrom zumindest teilweise zurück zur mindestens einen Destillationskolonne geführt wird. Ein Rücklauf kann dadurch eingestellt werden, dass am Kopf der jeweiligen Destillationskolonne ein Kondensator angebracht wird. In dem Kondensator wird der Brüdenstrom zumindest teilweise kondensiert und wieder der Destillationskolonne zugeführt. In den Fällen, in denen ein solcher Rücklauf eingestellt wird, beträgt das Rücklaufverhältnis dabei bevorzugt kleiner 1 bis 100, weiterhin bevorzugt 1 bis 50, besonders bevorzugt 1 bis 30. Unter einem Rücklaufverhältnis wird allgemein und im Sinne dieser Erfindung das Verhältnis aus dem Anteil des aus der Kolonne entnommenen Massenstroms (kg/h), der wieder auf die Kolonne in flüssiger Form zurückgeführt wird (Rücklauf) zu dem Anteil dieses Massenstroms (kg/h), der in flüssiger Form oder gasförmiger Form von der jeweiligen Kolonne abgeführt wird, verstanden.

Die Temperatur und der Druck in der mindestens einen Destillationskolonne bestimmen sich nach der gewünschten Trennaufgabe und damit anhand des eingesetzten Kohlenwasserstoffstroms. Das Auffinden der richtigen Temperatur und des richtigen Drucks stellt für den Fachmann kein Problem dar. Temperatur und Druck wird der Fachmann recht einfach anhand der relativen Flüchtigkeit bzw. anhand der zu bewältigenden Trennaufgabe ermitteln können.

Destillationsverfahren sind in aller Regel recht energieintensive Verfahren, bei denen über den Sumpf Wärmeenergie eingetragen wird, um die Trennaufgabe zu bewältigen. Gleichzeitig wird der abgetrennte Brüden gekühlt, um ihn zumindest teilweise zu kondensieren. Es ist vorteilhaft beim erfindungsgemäßen Verfahren wärmeintegrative Maßnahmen zu ergreifen, um Energie innerhalb des Systems zurückzugewinnen und nutzbar zu machen. Geeignete Maßnahmen zur Wärmeintegration sind die Brüdenverdichtung und der Einsatz einer Wärmepumpe.

Eine mögliche Maßnahme zur Wärmeintegration ist die Brüdenverdichtung. Dabei wird der am Kopf entnommene Brüdenstrom zumindest teilweise verdichtet. Der Druck des Brüdenstroms wird dabei erhöht. Durch das Verdichten wird zusätzliche Energie in das System gebracht. Das Verdichten zumindest eines Teils des Brüdenstroms kann auf jede beliebige, dem Fachmann bekannte Art erfolgen. So kann die Verdichtung zum Beispiel mechanisch und einstufig oder mehrstufig durchgeführt werden. Einstufig bedeutet in diesem Zusammenhang, dass eine Verdichtung von einem auf ein anderes Druckniveau stattfindet. Mehrstufig bedeutet, dass erst auf ein Druckniveau X und dann von X auf das Druckniveau Y verdichtet wird. Bei einer mehrstufigen Verdichtung können mehrere Verdichter gleicher Bauart oder Verdichter unterschiedlicher Bauart eingesetzt werden. Eine mehrstufige Verdichtung kann bevorzugt mit einer Verdichtermaschine oder mit mehreren Verdichtermaschinen erfolgen. Der Einsatz einer einstufigen Verdichtung oder einer mehrstufigen Verdichtung ist vom Verdichtungsverhältnis und somit davon abhängig, auf welchen Druck der Brüdenteilstrom verdichtet werden soll.

Als Verdichter im erfindungsgemäßen Verfahren, insbesondere zum Verdichten des Brüdenstroms, eignet sich jeder beliebige, dem Fachmann bekannte Verdichter, bevorzugt mechanische Verdichter, mit dem sich Gasströme verdichten lassen. Geeignete Verdichter sind zum Beispiel ein- oder mehrstufige Getriebeturboverdichter, Kolbenverdichter, Schraubenverdichter, Zentrifugalverdichter oder Axialverdichter.

Nach dem Verdichten wird der verdichtete Brüdenstrom zu einem Wärmetauscher geführt, wo er Wärmeenergie auf einen anderen Strom überträgt. Es wäre beispielsweise möglich, Wärmeenergie auf den Strom im Sumpfverdampfer übertragen und damit die Destillationskolonne zu beheizen. Die Phrase "Übertragung von Energie" bedeutet erfindungsgemäß insbesondere Beheizung, also Übertragung von Energie in Form von Wärme.

Eine andere Maßnahme zur Wärmeintegration ist der Einsatz einer Wärmepumpe. Dabei wird der am Kopf der mindestens einen Destillationskolonne entnommene Brüdenstrom dazu verwendet Wärmeenergie auf einen Wärmeträger zu übertragen. Nach der Energieübertragung weist der Wärmeträger vorzugsweise eine erhöhte Temperatur und/oder einen erhöhten Druck auf.

Die Übertragung von Energie kann nach dem Fachmann bekannten Verfahren oder mit dem Fachmann bekannten Wärmetauschern durchgeführt werden. Geeignete Verdampfer, die als Wärmetauscher eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Neben den genannten kann aber auch jede beliebige andere, dem Fachmann bekannte Verdampferbauart, die sich zum Einsatz an einer Destillationskolonne eignet, eingesetzt werden. Der Wärmetauscher kann in diesem Fall auch der Kondensator zur Kondensation des Brüdenstroms sein. Das hat den Vorteil, dass kein zusätzlicher Kondensator eingebaut werden muss.

Als Wärmeträger kann jedes dem Fachmann geläufige Arbeitsmedium genutzt werden. Der Wärmeträger wird vorzugsweise aus der Gruppe bestehend aus Wasser; Alkoholen; Alkohol-Wasser-Mischungen; Salz-Wasser-Lösungen; Ammoniak; Mineralölen, wie zum Beispiel Dieselöle; Thermalölen, wie zum Beispiel Silikonöle; biologischen Ölen, wie zum Beispiel Limonen; und aromatischen oder aliphatischen Kohlenwasserstoffen, wie zum Beispiel Dibenzyltoluol ausgewählt, weiterhin bevorzugt sind Wasser, Methanol, Ethanol, Propanol, n-Pentan, n-Butan, n-Hexan, n-Propan oder Ammoniak, besonders bevorzugt ist Wasser.

Nach der erwähnten Energieübertragung wird der Wärmeträger zumindest teilweise verdichtet, wodurch ein verdichteter Wärmeträger entsteht, der einen höheren Druck aufweist als der Wärmeträger vor der Verdichtung.

Das Verdichten des mindestens einen Teils des Wärmeträgers kann auf jede beliebige, dem Fachmann bekannte Art erfolgen. So kann die Verdichtung, wie oben bereits definiert, zum Beispiel mechanisch und einstufig oder mehrstufig durchgeführt werden. Als Verdichter im erfindungsgemäßen Verfahren eignet sich jeder beliebige, dem Fachmann bekannte Verdichter, bevorzugt mechanische Verdichter, mit dem sich Gasströme verdichten lassen. Geeignete Verdichter sind zum Beispiel ein- oder mehrstufige Getriebeturboverdichter, Kolbenverdichter, Schraubenverdichter, Zentrifugalverdichter oder Axialverdichter.

Im nächsten Schritt wird Energie vom verdichteten Wärmeträger auf einen zu erwärmenden Strom im System übertragen, vorzugsweise im Sumpfverdampfer, um damit eine Destillationskolonne zu beheizen.

Im Unterschied zur Brüdenverdichtung, bei der der am Kopf der Destillationskolonne anfallende Brüden verdichtet und zur Übertragung von Wärmeenergie eingesetzt wird, wird beim Einsatz einer Wärmepumpe also ein Wärmeträger zwischengeschaltet. Das Prinzip ist jedoch gleich: Energie wird an einer Stelle im Verfahren eingesammelt und an anderer Stelle im Verfahren eingesetzt.

Das vorliegende Verfahren wird für die Auftrennung eines Raffinat-2-Stroms eingesetzt. Dabei besteht die Abtrenneinheit vorzugsweise aus mindestens zwei Destillationskolonnen DK1 und DK2, wobei die mindestens zwei unterschiedlichen Einlässe für den Kohlenwasserstoffstrom an der ersten Destillationskolonne DK1 vorhanden sind. Das Raffinat 2, das zur ersten Destillationskolonne DK1 geleitet wird, wird in der Destillationskolonne DK1 in mindestens zwei Ströme aufgetrennt, d. h. in mindestens einen Brüdenstrom BS1, der zumindest 1-Buten und Isobutan umfasst und am Kopf der DK1 entnommen wird, und in mindestens einen Sumpfstrom, der zumindest 1-Buten und 2-Buten umfasst und am Sumpf der DK1 entnommen wird. Der Brüdenstrom BS1 kann am Kopf der Destillationskolonne auch in Form von mehreren Teilströmen BS1(n), wobei n eine ganze Zahl und gleich der Anzahl der Teilströme ist, entnommen werden. Gleiches gilt auch für den Sumpfstrom. Im Sumpf der ersten Destillationskolonne DK1 liegt vorzugsweise eine Temperatur im Bereich von 40 bis 110 °C, vorzugsweise 50 bis 100 °C vor.

Im Folgenden werden der Druck und die Temperatur des Brüdenstroms BS1 angegeben. Dies bezieht sich insbesondere auf den Druck und die Temperatur des mindestens einen Brüdenstroms BS1, wenn er der Destillationskolonne DK1 entnommen wird. Der Druck des Brüdenstroms BS1 liegt insbesondere im Bereich von 6 bis 15 bar absolut, vorzugsweise im Bereich von 7,5 bis 13 bar absolut. Die Temperatur des Brüdenstroms BS1 liegt insbesondere im Bereich von 45 °C bis 120 °C, bevorzugt im Bereich von 48 °C bis 100 °C, weiterhin bevorzugt im Bereich von 50 °C bis 90 °C, weiterhin bevorzugt im Bereich von 55 °C bis 80 °C, besonders bevorzugt im Bereich von 60 °C bis 80 °C.

Die Entnahme von mindestens einem Brüdenstroms BS1, der zumindest 1-Buten und Isobutan umfasst, am Kopf der Destillationskolonne DK1 bedeutet im Rahmen der vorliegenden Erfindung insbesondere, dass der mindestens eine Brüdenstrom BS1 als Kopfstrom oder als Seitenabzug oberhalb der Einbauten in der Destillationskolonne DK1 entnommen wird.

Die Entnahme des mindestens einen Sumpfstroms, der zumindest 1-Buten und 2-Buten umfasst, am Sumpf der Destillationskolonne DK1 bedeutet im Rahmen der vorliegenden Erfindung insbesondere, dass der mindestens eine Sumpfstrom direkt am Sumpf oder am unteren Boden der Destillationskolonne DK1 entnommen wird.

Die Destillationskolonne DK1 wird vorzugsweise mit Rücklauf betrieben. Rücklauf bedeutet, dass der am oberen Ende der Destillationskolonne DK1 entnommene Brüdenstrom BS1 mindestens teilweise wieder der Destillationskolonne DK1 zugeführt wird. In den Fällen, in denen ein solcher Rücklauf eingestellt wird, beträgt das Rücklaufverhältnis dabei bevorzugt 2 bis 30, bevorzugter 5 bis 20, besonders bevorzugt 8 bis 15.

Der Brüdenstrom aus der ersten Destillationskolonne DK1 wird zur zweiten Destillationskolonne DK2 geführt. Wärmeintegrative Maßnahmen wie die Brüdenverdichtung oder der Einsatz einer Wärmepumpe sind hier möglich, wobei Energie durch zumindest teilweise Kondensation des Brüdenstroms BS1 eingesammelt und zur Beheizung von DK1 und/oder DK2 eingesetzt werden kann. Sind die Mengen an Isobuten im zur Destillationskolonne DK2 geführten Strom zu hoch, um die Spezifikation des 1-Buten-Produkts aus der DK2 zu erfüllen, kann eine zusätzliche MTBE- oder ETBE-Synthese zwischen den Destillationskolonnen DK1 und DK2 angeordnet sein. Dafür wird der Strom zu einer MTBE- oder ETBE-Synthese geführt, das enthaltene Isobuten zumindest teilweise zu MTBE oder ETBE umgesetzt und anschließend das gebildete MTBE oder ETBE abgetrennt.

Die MTBE- oder ETBE-Synthese ist dem Fachmann grundsätzlich bekannt. Für die Herstellung von MTBE oder ETBE aus isobutenhaltigen Strömen können insbesondere saure lonenaustauschharze (Sulfonsäuregruppen) als heterogene Katalysatoren eingesetzt. Die MTBE- oder ETBE-Synthese kann in einem oder mehreren in Reihe geschalteten Reaktoren stattfinden. Der Katalysator wird vorzugsweise als Festbettkatalysator eingesetzt. Da die Bildung von MTBE oder ETBE eine Gleichgewichtsreaktion darstellt, kann es zweckmäßig sein mindestens eine Reaktivdestillationskolonne einzusetzen, in der gleichzeitig die Reaktion und die Abtrennung des MTBE bzw. des ETBE erfolgen. Bei der Reaktivdestillation sollte ein Druck im Bereich von 3 bis 15 bar und eine Temperatur in der Reaktionszone von 55 bis 75 °C vorliegen. Nach der Synthese werden MTBE oder ETBE abgetrennt, vorzugsweise mittels Destillation. Auch dieses Verfahren ist dem Fachmann bekannt. Der dann erhaltene Strom, der weniger Isobuten enthält, kann dann zur Destillationskolonne DK2 geführt werden.

In der zweiten Destillationskolonne DK2 wird der zugeführte Kohlenwasserstoffstrom, hier der Brüdenstrom aus der DK1, der zumindest Isobutan und 1-Buten umfasst, in mindestens einen Brüdenstrom BS2, der zumindest Isobutan umfasst und am Kopf der DK2 entnommen wird, und in mindestens einen Produktstrom, der zumindest 1-Buten umfasst und am Sumpf der DK2 entnommen wird, aufgetrennt. Die Destillationskolonne DK2 kann ebenfalls mindestens zwei unterschiedliche Einlässe für den einzuspeisenden Kohlenwasserstoffstrom aus der DK1 aufweisen, wobei die Einlässe vom Kopf der Destillationskolonne DK2 betrachtet unterhalb voneinander angeordnet sind.

Als Destillationskolonne DK2 für die Auftrennung des Raffinat-2-Stroms kann jede beliebige, dem Fachmann bekannte Destillationskolonne eingesetzt werden. Bevorzugt enthält die Destillationskolonne DK2 Einbauten. Geeignete Einbauten sind zum Beispiel Böden, unstrukturierte Packungen (Füllkörper) oder strukturierte Packungen. Als Böden werden üblicherweise Glockenböden, Siebböden, Ventilböden mit festen oder beweglichen Ventilen, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel, SuperRinge/SuperRinge Plus oder Intalox^{®}-Sättel verwendet. Strukturierte Packungen werden zum Beispiel unter dem Handelsnamen Mellapak^{®} der Firma Sulzer vertrieben. Neben den genannten Einbauten sind dem Fachmann weitere geeignete Einbauten bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Destillationskolonne DK2 möglichst gering bleibt und somit die mechanische Leistung des Verdichters und die Temperatur des zu verdampfenden Stroms gering bleibt.

In einer besonders bevorzugten Ausführungsform der vorliegenden Erfindung umfasst die zweite Destillationskolonne DK2 eine Vielzahl von Böden, vorzugsweise zwischen 150 und 300 Böden, weiterhin bevorzugt zwischen 170 und 220 Böden.

Die Entnahme mindestens einen Brüdenstroms BS2, der zumindest Isobutan umfasst, am Kopf der Destillationskolonne DK2 bedeutet im Rahmen der vorliegenden Erfindung insbesondere, dass der mindestens eine Brüdenstrom BS2 als Kopfstrom oder als Seitenabzug oberhalb der Einbauten in der Destillationskolonne DK2 entnommen wird.

Die Entnahme des mindestens einen Produktstroms, der zumindest 1-Buten umfasst, am Sumpf der Destillationskolonne DK2 bedeutet im Rahmen der vorliegenden Erfindung insbesondere, dass der mindestens eine Produktstrom direkt am Sumpf oder am unteren Boden der Destillationskolonne DK2 entnommen wird. Der Produktstrom enthält vorzugsweise mindestens 99 Gew.-% 1-Buten, weiterhin bevorzugt mindestens 99,5 Gew.-% 1-Buten, besonders bevorzugt mindestens 99,6 Gew.-% 1-Buten. Das 1-Buten ist das Zielprodukt des vorliegenden Verfahrens, weshalb der Produktstrom aus dem Verfahren ausgeschleust wird. Das 1-Buten kann beispielsweise als Co-Monomer bei der Herstellung von Polyethylen eingesetzt werden.

Im Sumpf der zweiten Destillationskolonne DK2 liegt während des erfindungsgemäßen Verfahrens vorzugsweise eine Temperatur im Bereich von 30 bis 100 °C, vorzugsweise 45 bis 80 °C vor. Weiterhin bevorzugt liegt am Kopf der zweiten Destillationskolonne DK2 ein Druck im Bereich von 3 bis 12 bar absolut, vorzugsweise 5 bis 10 bar absolut vor.

Die Destillationskolonne DK2 kann weiterhin mit Rücklauf betrieben werden. Rücklauf bedeutet, dass der am oberen Ende der Destillationskolonne DK2 entnommene Brüdenstrom BS2 mindestens teilweise wieder der Destillationskolonne DK2 zugeführt wird. In den Fällen, in denen ein solcher Rücklauf eingestellt wird, beträgt das Rücklaufverhältnis dabei bevorzugt 10 bis 100, besonders bevorzugt 30 bis 50.

Die vorliegende Erfindung wird nachfolgend anhand von Abbildungen erläutert. Die Abbildungen dienen der Veranschaulichung, sind aber nicht einschränkend zu verstehen.

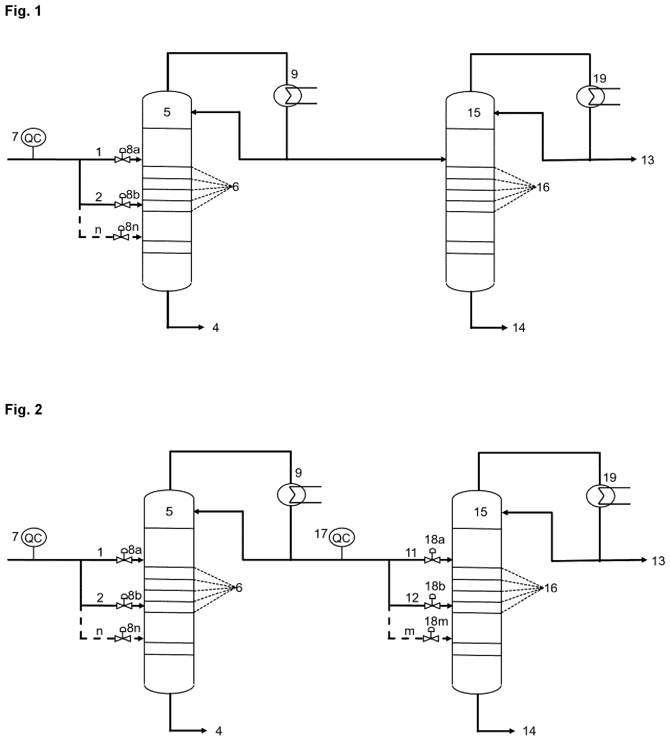

Fig. 1 zeigt eine Ausführungsform mit zwei Destillationskolonnen DK1 (5) und DK2 (15), die eine Vielzahl von Einbauten (6, 16) aufweisen. Die Einbauten (6, 16) sind nur schematisch gezeigt, d. h. der Aufbau kann in der Realität abweichen. Zudem ist die Anzahl in der industriellen Praxis oft deutlich höher. Die Einbauten (6, 16) sind vom Kopf der Kolonne untereinander angeordnet. Im vorliegenden Fall sind die Einbauten Böden. Das aufzutrennende Raffinat 2 kann über die Leitung 1, die Leitung 2 oder eine ggf. zusätzliche Leitung n bzw. über die zugehörigen Einlässe in die Destillationskolonne DK1 (5) geleitet werden. Die gestrichelte Leitung n deutet an, dass noch weitere Einlässe vorhanden sein können. Welche dieser Leitungen zu wählen ist, wird durch Analyse des Raffinat 2 über die entsprechende Analyseeinheit QC (7) bestimmt. Die Zuleitung des Raffinat 2 über einen der Einlässe (1, 2, n) wird mithilfe Ventile (8a, 8b, 8n) eingestellt. Am Sumpf der Kolonne DK1 (5) der Sumpfstrom (4) abgenommen. Am Kopf fällt der Brüdenstrom an, der im Wärmetauscher (9) kondensiert wird. Ein Teil des kondensierten Brüdenstroms wird in die erste Destillationskolonne DK1 (5) und ein anderer Teil in die zweite Destillationskolonne DK2 (15). Dort findet eine weitere Destillation statt. Am Sumpf der Kolonne DK2 (15) der Sumpfstrom (14) abgenommen. Am Kopf fällt der Brüdenstrom an, der im weiteren Wärmetauscher (19) kondensiert wird. Ein Teil des kondensierten Brüdenstroms wird in die erste Destillationskolonne DK2 (15) und ein anderer Teil wird ausgeschleust. Die Wärmeintegration, bei der die Kondensationsenergie die an den Wärmetauschern (9, 19) anfällt wird dann zur Beizung eines oder beider Sümpfe der Destillationskolonnen DK1 und DK2 (5, 15) eingesetzt, was in der Abbildung nicht gezeigt ist.
Fig. 2 zeigt eine weitere erfindungsgemäße Ausführungsform, die weitgehend der Ausführungsform nach Fig. 1 entspricht. Der Unterschied zu Fig. 1 besteht darin, dass der Brüdenstrom aus der ersten Destillationskolonne DK1 (5) über die Leitung 11, die Leitung 12 oder eine ggf. zusätzliche Leitung m bzw. über die zugehörigen Einlässe in die Destillationskolonne DK2 (15) geleitet werden kann. Die gestrichelte Leitung m deutet an, dass noch weitere Einlässe vorhanden sein können. Welche dieser Leitungen zu wählen ist, wird durch Analyse des Raffinat 2 über die entsprechende Analyseeinheit QC (17) bestimmt. Die Zuleitung des Teils des kondensierten Brüdenstroms über einen der Einlässe (11, 12, m) wird mithilfe Ventile (18a, 18b, 18m) eingestellt.

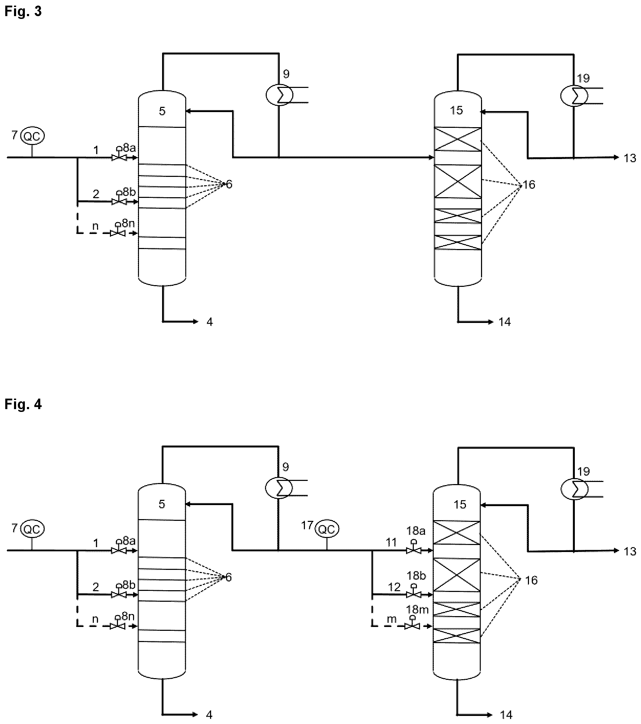

Fig. 3 zeigt eine weitere erfindungsgemäße Ausführungsform, die weitgehend der Ausführungsform nach Fig. 1 entspricht. Der Unterschied zu Fig. 1 besteht darin, dass als Einbauten (16) in der zweiten Destillationskolonne DK2 (15) Füllkörperschüttungen oder (strukturierte) Packungen vorhanden sind.
Fig. 4 zeigt eine weitere erfindungsgemäße Ausführungsform, die weitgehend der Ausführungsform nach Fig. 2 entspricht. Der Unterschied zu Fig. 2 besteht darin, dass als Einbauten (16) in der zweiten Destillationskolonne DK2 (15) Füllkörperschüttungen oder (strukturierte) Packungen vorhanden sind.

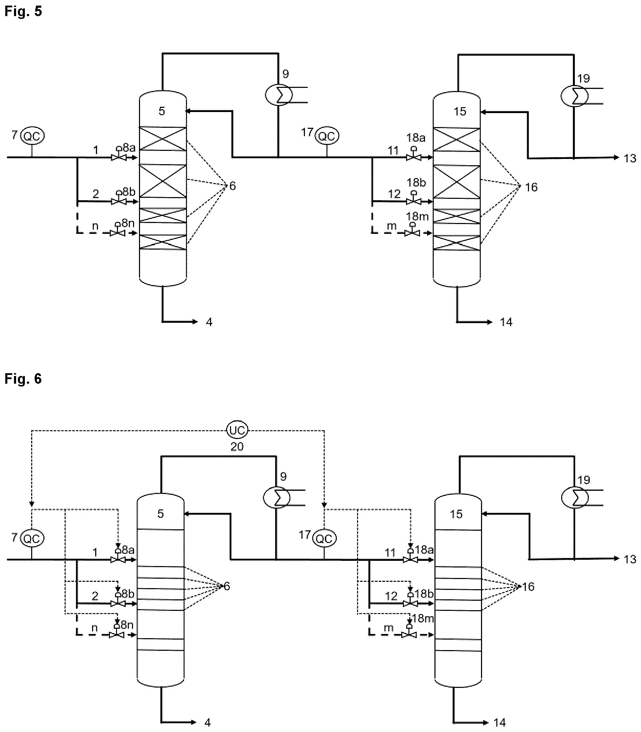

Fig. 5 zeigt eine weitere erfindungsgemäße Ausführungsform, die weitgehend der Ausführungsform nach Fig. 4 entspricht. Der Unterschied zu Fig. 4 besteht darin, dass als Einbauten (16) in der ersten Destillationskolonne DK1 (15) ebenfalls Füllkörperschüttungen oder (strukturierte) Packungen vorhanden sind.
Fig. 6 zeigt eine weitere Ausführungsform der vorliegenden Erfindung, die weitgehend der Ausführungsform nach Fig. 2 entspricht. Der Unterschied zu Fig. 2 besteht darin, dass die Analyseeinheiten (7, 17) mit einer Steuerungseinheit (UC) verbunden ist, über die die Analyseergebnisse ausgewertet werden und mithilfe der Ventile (8a, 8b, 8n, 18a, 18b, 18m) die Umschaltung auf einen anderen Einlass erfolgen kann.

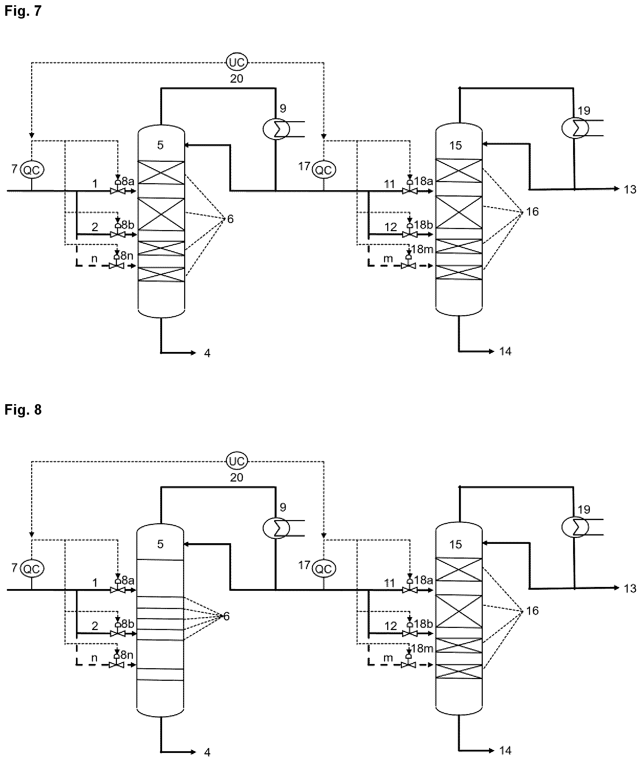

Fig. 7 zeigt eine weitere erfindungsgemäße Ausführungsform, die weitgehend der Ausführungsform nach Fig. 6 entspricht. Der Unterschied zu Fig. 6 besteht darin, dass als Einbauten (16) in der zweiten Destillationskolonne DK2 (15) Füllkörperschüttungen oder (strukturierte) Packungen vorhanden sind.
Fig. 8 zeigt eine weitere erfindungsgemäße Ausführungsform, die weitgehend der Ausführungsform nach Fig. 4 entspricht. Der Unterschied zu Fig. 7 besteht darin, dass als Einbauten (16) in der ersten Destillationskolonne DK1 (15) ebenfalls Füllkörperschüttungen oder (strukturierte) Packungen vorhanden sind.

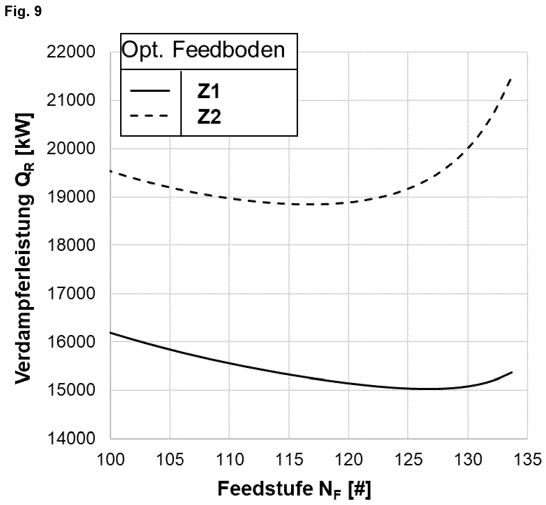

Fig. 9 zeigt ein Diagramm, bei dem die benötigte Verdampferleistung Q_{R} in Abhängig des Bodens (hier Feedstufe N_{F}) für beide Zusammensetzungen Z1 und Z2 aufgetragen ist. Erläuterungen dazu sind im nachfolgenden Beispiel zu finden.

### Beispiel

In diesem Beispiel wird eine Kolonne betrachtet, mit der 1-Buten und iso-Butan über Kopf aus einem Raffinat-2-Stroms abgetrennt werden. In diesem Beispiel werden zwei verschiedene Zusammensetzung (Z1 und Z2) des Raffinat 2 Stroms betrachtet, die z.B. die saisonalen Rohstoffschwankungen berücksichtigen sollen. In Tabelle 1 sind die verschiedenen Feed Zusammensetzungen dargestellt.

**Tabelle 1: Raffinat 2-Zusammensetzungen**

| | | **Z1** | **Z2** |
|---|---|---|---|
| | | x [wt%] | x [wt%] |
| i-Butan | | 7,5 | 7,5 |
| 1-Buten | | 45,5 | 35,5 |
| n-Butan | | 22,5 | 32,5 |
| 2-Buten | t-Buten | 16 | 16 |
| | c-Buten | 8,5 | 8,5 |

Die Rechnungen wurden mit Aspen Plus^{®} Version 10 und einem angepassten Stoffdatenmodell, welches auf betrieblich validierten Stoffdaten basiert, durchgeführt. Es wurde eine Kolonne mit 140 theoretischen Trennstufen (200 Böden mit Bodenwirkungsgrad von 0,7) berücksichtigt. Der n-Butananteil wurde auf 800 ppm im Kopf konstant gehalten. Fig. 5 zeigt das resultierende Diagramm, bei dem die benötigte Verdampferleistung Q_{R} in Abhängig des Bodens (hier Feedstufe N_{F}) für beide Zusammensetzungen Z1 und Z2 aufgetragen ist. Am niedrigsten Punkt in der jeweiligen Kurve ist der Energiebedarf am geringsten. Tabelle 2 zeigt eine Sensitivitätsanalyse, die den Zusammenhang zwischen dem Boden und der benötigten Wärmemenge in Megawatt (MW) des Verdampfers (QR) in Abhängigkeit der Zusammensetzung des Feeds (Z1 und Z2) darstellt. Bei einem hohen 1-Butenanteil im Feed (Z1) ist es vorteilhaft den Feed auf die 126. Trennstufe zu fahren. Der Verdampfer benötigt 0,13 MW weniger als wenn das Raffinat mit der Zusammensetzung Z1 auf die 119. Trennstufe gefahren wird. Für hohe n-Butananteile im Feed wie in Zusammensetzung Z2 wird insgesamt eine größere Wärmemenge benötigt. Ferner verschiebt sich der optimale Boden. Wohingegen bei Zusammensetzung Z1 der Boden 126 energetisch vorteilhaft ist, ist bei Zusammensetzung Z2 der Boden 119 energetisch bevorzugt zu wählen. Demzufolge resultiert eine Umstellung des Bodens in einer Energieeinsparung von 0,49 MW. Diese Energieeinsparungen resultieren in einer CO₂- und Betriebskosteneinsparung.

**Tabelle 2: Ergebnisse der Sensitivitätsanalyse**

| | **Z1** | **Z2** |
|---|---|---|
| **Trennstufe** | QR [MW] | QR [MW] |
| 119 | 15,15 | 18,87 |
| 126 | 15,02 | 19,36 |

## Patentansprüche

1. Verfahren zur Destillation eines Raffinat-2-Stroms als Feedstrom, in einer Abtrenneinheit, die mindestens zwei Destillationskolonnen DK1 und DK2 umfasst, wobei
die Destillationskolonne DK1 und die DK2 jeweils einen Kopf am oberen Ende der Kolonne und einen Sumpf am unteren Ende der Kolonne umfassen, wobei am Kopf jeweils ein Brüdenstrom und am Sumpf jeweils ein Sumpfstrom anfallen und wobei die Destillationskolonne DK2 mit einem Teil des Brüdenstroms aus der DK2 gespeist wird;
die Brüdenströme von DK1 und DK2 zumindest teilweise kondensiert werden und die dabei anfallende Kondensationsenergie von mindestens einem der Brüdenströme von DK1 oder DK2 mittels Brüdenverdichtung oder mittels einer Wärmepumpe zur Beheizung von zumindest einer der Destillationskolonnen DK1 oder DK2 eingesetzt werden;
zumindest die Destillationskolonne DK1 mindestens zwei unterschiedliche Einlässe für den Feedstrom aufweist, wobei die Einlässe vom Kopf der Destillationskolonne betrachtet unterhalb voneinander angeordnet sind;
der Feedstrom vor dem Einleiten auf seine Zusammensetzung analysiert wird; und
in Abhängigkeit von der Zusammensetzung des Feedstroms einer der mindestens zwei Einlässe für die Zufuhr des Feedstroms gewählt wird.

2. Verfahren nach einem der vorhergehenden Ansprüche, wobei die Destillationskolonne DK1 Einbauten, wie Füllkörperschüttungen, (strukturierte) Packungen oder Böden umfasst, vorzugsweise zwischen 2 und 300 Einbauten, weiterhin bevorzugt zwischen 2 und 250 Einbauten, besonders bevorzugt zwischen 2 bis 220 Einbauten.

3. Verfahren nach Anspruch 2, wobei die Einbauten innerhalb der DK1 vom Kopf der Destillationskolonne betrachtet unterhalb voneinander angeordnet sind und die mindestens zwei Einlässe auf Höhe unterschiedlicher Einbauten vorhanden sind.

4. Verfahren nach einem der vorhergehenden Ansprüche, wobei sich während des Verfahrens die Zusammensetzung des Raffinat-2-Stroms ändert und auf einen anderen Einlass umgeschaltet werden muss.

5. Verfahren nach Anspruch 4, wobei die Umschaltung des Raffinat-2-Stroms auf einen anderen Einlass automatisch oder manuell erfolgt.

6. Verfahren nach Anspruch 5, wobei die Umschaltung automatisch darüber erfolgt, dass die Analyseergebnisse mittels einer Steuereinheit, vorzugsweise mittels eines Computers ausgewertet werden und bei Über- oder Unterschreiten eines Grenzwerts in der Zusammensetzung des Raffinat-2-Stroms die Umschaltung auf einen anderen Einlass erfolgt.

7. Verfahren nach einem der vorhergehenden Ansprüche, wobei die Analyse der Zusammensetzung des Raffinat-2-Stroms mittels Raman-Spektroskopie oder mittels Gaschromatographie durchgeführt wird.

8. Verfahren nach einem der vorhergehenden Ansprüche, wobei das Verfahren kontinuierlich oder in Batch-Fahrweise, vorzugsweise kontinuierlich betrieben wird.

9. Verfahren nach einem der vorhergehenden Ansprüche, wobei mindestens eine der Destillationskolonnen DK! Und DK2 mit Rücklauf betrieben wird und das Rücklaufverhältnis kleiner 1 bis 100, weiterhin bevorzugt 1 bis 50, besonders bevorzugt 1 bis 30 beträgt.

10. Verfahren nach einem der vorhergehenden Ansprüche, wobei die Zusammensetzung des Raffinat-2-Stroms s in vorab definierten zeitlichen Abständen kontinuierlich während des gesamten Verfahrens analysiert wird.

11. Verfahren nach einem der vorhergehenden Ansprüche, wobei die Destillationskolonne DK2 mindestens zwei unterschiedliche Einlässe für den einzuspeisenden Kohlenwasserstoffstrom aus der DK1 aufweist und die Einlässe vom Kopf der Destillationskolonne DK2 betrachtet unterhalb voneinander angeordnet sind.

12. Verfahren nach Anspruch 11, wobei die Destillationskolonne DK2 Einbauten, wie Füllkörperschüttungen, (strukturierte) Packungen oder Böden umfasst, vorzugsweise zwischen 2 und 300 Einbauten, weiterhin bevorzugt zwischen 2 und 250 Einbauten, besonders bevorzugt zwischen 2 bis 220 Einbauten.

13. Verfahren nach Anspruch 12, wobei die Einbauten innerhalb der DK2 vom Kopf der Destillationskolonne betrachtet unterhalb voneinander angeordnet sind und die mindestens zwei Einlässe auf Höhe unterschiedlicher Einbauten vorhanden sind.
